# DEMANDE DE BREVET EUROPEEN

(11) **EP 1 236 999 A1**
(43) Date de publication de la demande: **04.09.2002**
(21) Numéro de dépôt: 02290179.7
(22) Date de dépôt: 25.01.2002
(51) Int. Cl.: G01N 33/00, G01N 33/22, G01N 33/28

(54) **Procédé et dispositif de détection d'hydrocarbures dans un gaz**

(30) Priorité: 06.02.2001 FR 0101596
(71) Demandeur: L'AIR LIQUIDE, Société Anonyme à Directoire et Conseil de Surveillance pour l'Etude et l'Exploitation des, 75321 Paris Cédex 07 (FR)
(72) Inventeur: Bryselbout, Francis, 78320 Le Mesnil St Denis (FR)
(74) Mandataire: Ducreux, Marie

(57) **Abrégé**

L'invention concerne un procédé de détection d'hydrocarbures autres que le méthane dans un gaz comportant majoritairement ou essentiellement de l'oxygène, ainsi que du méthane et lesdits hydrocarbures autres que le méthane, ledit procédé comportant :
- une étape de détection de l'ensemble des hydrocarbures dans ledit gaz, fournissant une première valeur d'ensemble d'hydrocarbures,
- une étape de combustion des hydrocarbures autres que le méthane,
- une étape de détection du méthane dans ledit gaz, fournissant une deuxième valeur,
- une étape de calcul de la quantité d'hydrocarbures autres que le méthane par différence entre la première valeur et la deuxième valeur.

L'invention concerne également un dispositif pour mettre en oeuvre ce procédé.

## Description

### DOMAINE TECHNIQUE ET ART ANTERIEUR

L'invention concerne le domaine de la détection d'hydrocarbures autres que le méthane dans un gaz comportant ou comprenant essentiellement de l'oxygène, et notamment dans un gaz comportant au moins 95 % d'oxygène avec des impuretés, notamment des impuretés hydrocarbures, lesdites impuretés hydrocarbures étant par exemple présentes à moins de 200 ppm.

L'invention concerne également les unités de production des gaz de l'air. En effet, la présence d'hydrocarbures non méthanes dans le bain d'oxygène liquide des vaporisateurs de ces unités de production entraîne un risque d'explosion lors du dépassement de certaines limites de concentrations. Ces limites sont définies dans les consignes d'exploitation des unités de production.

Les techniques actuellement utilisées pour identifier et détecter ces hydrocarbures sont d'une part la chromatographie, et d'autre part la spectrométrie infrarouge (FTIR).

La chromatographie est une technique qui est utilisée depuis très longtemps. Elle est séquentielle et ne permet de détecter et de mesurer que quelques hydrocarbures présélectionnés. L'investissement et le coût de la maintenance du chromatographe sont importants.

La spectrométrie infrarouge (FTIR) est une technique qui, bien que plus performante que la chromatographie, n'est pas totalement globale, car elle nécessite un examen du spectre pour rechercher une impureté qui n'aurait pas été sélectionnée. L'appareillage nécessite en outre un investissement important.

Il se pose donc le problème de trouver une nouvelle méthode et un nouveau dispositif qui permettent une mesure et une détection continues d'hydrocarbures autres que le méthane, dans un gaz comportant essentiellement de l'oxygène.

Il se pose également le problème de trouver une méthode et un dispositif qui soient plus simples à mettre en oeuvre, et moins coûteux, que les techniques et les dispositifs antérieurs.

Il se pose également le problème de trouver une méthode et un dispositif qui permettent une mesure et une détection continues d'hydrocarbures autres que le méthane, dans un gaz comportant essentiellement de l'oxygène, lesdits hydrocarbures autres que le méthane étant présent, par rapport au méthane, dans une proportion de l'ordre de quelques pour cent.

Il se pose également le problème d'assurer la sécurité d'unités de production des gaz de l'air, comportant un vaporisateur, par la détection globale continue des hydrocarbures gazeux non méthanes à une concentration de quelques ppm (par exemple : moins de 5 ppm) dans l'oxygène du vaporisateur, qui peut lui-même contenir de méthane, par exemple à environ 50 ppm.

### EXPOSE DE L'INVENTION

L'invention a tout d'abord pour objet un procédé de détection d'hydrocarbures autres que le méthane dans de l'oxygène ou dans un gaz comportant ou comportant essentiellement de l'oxygène (l'oxygène étant en outre mélangé avec du méthane et lesdits hydrocarbures autres que le méthane), ledit procédé comportant:
- une étape de détection de l'ensemble des hydrocarbures dans ledit oxygène ou dans ledit gaz, fournissant une première valeur d'ensemble d'hydrocarbures,
- une étape de combustion des hydrocarbures autres que le méthane,
- une étape de détection du méthane dans ledit oxygène ou dans ledit gaz, fournissant une deuxième valeur, suivie de préférence par,
- une étape de calcul de la quantité d'hydrocarbures autres que le méthane par différence entre la première valeur et la deuxième valeur.

Un tel procédé permet de réaliser une mesure en continu des hydrocarbures autres que le méthane.

Les étapes de détection peuvent être réalisées par un détecteur à ionisation de flamme. On réalise ainsi un système de détection qui est simple d'utilisation, qui fonctionne en continu, qui est précis, moins coûteux, et qui nécessite moins de maintenance que les systèmes connus.

Selon un mode de réalisation, les hydrocarbures autres que le méthane sont brûlés dans un catalyseur.

De l'hydrogène peut être en outre mélangé au gaz à analyser, de sorte que le mélange présente un rapport H2 :O2 proche ou du même ordre que le rapport H2 :O2 dans l'air.

La température du catalyseur est de préférence telle que moins de 5% du méthane contenu dans le gaz est brûlé.

L'invention concerne également un procédé de détection d'hydrocarbures autres que le méthane dans un bain d'oxygène liquide d'un vaporisateur d'une unité de production de gaz de l'air, comportant :
- un prélèvement d'oxygène liquide dans ledit bain,
- une vaporisation dudit oxygène liquide, produisant un gaz vaporisé,
- un procédé de détection d'hydrocarbures autres que le méthane dans ledit gaz vaporisé, tel que décrit ci-dessus.

De préférence, le prélèvement est réalisé à l'aide d'une tuyauterie d'une pompe de remontée de liquide ou sur une prise de type ascenseur, ce qui permet d'échantillonner le gaz de manière rapide, et donc d'analyser à chaque instant un échantillon assez représentatif du mélange à analyser au même instant.

L'invention concerne également un dispositif de détection d'hydrocarbures autres que le méthane dans un gaz comportant majoritairement ou essentiellement de l'oxygène, ainsi que du méthane et lesdits hydrocarbures autres que le méthane, spécialement adapté pour mettre en oeuvre un procédé tel que décrit ci-dessus, ledit dispositif comportant :
- des moyens de détection de l'ensemble des hydrocarbures dans ledit gaz, fournissant une première valeur d'ensemble d'hydrocarbures,
- des moyens de combustion des hydrocarbures autres que le méthane,
- des moyens de détection du méthane, et de préférence,
- des moyens pour, ou spécialement programmés pour, calculer la quantité d'hydrocarbures autres que le méthane, par différence entre la première et deuxième valeur.

L'invention concerne également un dispositif de détection d'hydrocarbures autres que le méthane dans un bain d'oxygène liquide d'un vaporisateur d'une unité de fabrication de gaz de l'air, comportant :
- des moyens de prélèvement d'oxygène liquide dans ledit bain,
- des moyens pour vaporiser ledit oxygène liquide, produisant un gaz vaporisé,
- un dispositif de détection tel que décrit ci-dessus.

Des moyens peuvent en outre être prévus pour déclencher une alarme lorsque la concentration ou le taux d'hydrocarbures autres que le méthane, dans ledit gaz vaporisé, dépasse une certaine valeur limite.

### BREVE DESCRIPTION DES FIGURES

Les caractéristiques et avantages de l'invention apparaîtront mieux à la lumière de la description qui va suivre. Cette description porte sur les exemples de réalisation, donnés à titre explicatif et non limitatif, en se référant à des dessins annexés sur lesquels:
- la figure 1 représente un exemple de réalisation de l'invention,
- la figure 2 représente la structure d'un détecteur pouvant être utilisé dans le cadre de la présente invention,
- la figure 3 représente un test de réponse d'hydrocarbures non méthane dans de l'oxygène,
- les figures 4A à 6B représentent différents essais réalisés sur des mélanges d'oxygène et d'hydrocarbure,
- les figures 7 et 8 représentent un dispositif de prélèvement et d'analyse à partir d'un bain d'oxygène liquide.

### DESCRIPTION DETAILLEE DE MODES DE REALISATION DE L'INVENTION

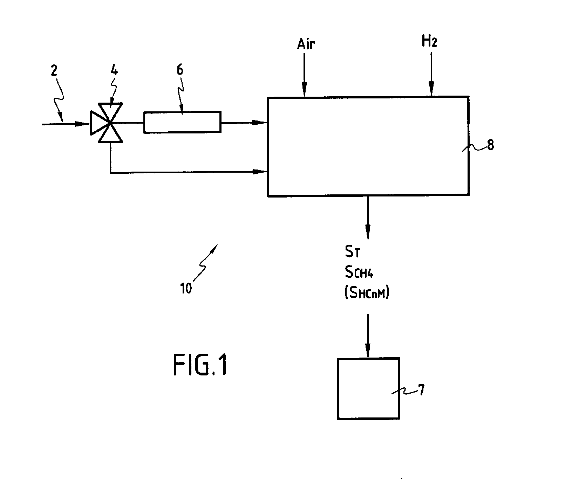

Un exemple de réalisation de l'invention est donné sur la figure 1.

Sur cette figure, la référence 2 désigne une arrivée d'un gaz à mesurer, comportant ou comprenant de l'oxygène ou essentiellement de l'oxygène, et contenant des impuretés hydrocarbures, par exemple à moins de environ 200 ppm, par exemple 100 ppm ou 50ppm ou à un taux de l'ordre de quelques dizaines de ppm.

Parmi les impuretés hydrocarbures, il peut y avoir d'une part du méthane et d'autre part des hydrocarbures gazeux non méthane. Selon un exemple, le méthane est présent à un taux de environ 50 ppm, et les hydrocarbures non gazeux le sont à un taux de l'ordre de 5 ppm.

D'autres impuretés peuvent en outre être présentes, notamment de l'azote ou de l'argon, mais le gaz comporte essentiellement de l'oxygène à au moins 95 % et de préférence au moins 99% ou 99,5%.

Dans le cas où seuls des hydrocarbures sont présents, à un taux de 200 ppm, le gaz comporte 99,98% d'oxygéne.

La référence 10 désigne un système d'analyse selon l'invention.

Le gaz à mesurer peut, par une vanne 4, être soit envoyé sur un catalyseur 6, qui permet de brûler les hydrocarbures gazeux non méthane (HCnM, ou mesure en mode CH4), puis sur un détecteur 8, soit être envoyé directement sur le détecteur 8. Dans le deuxième cas, tous les hydrocarbures sont détectés (hydrocarbures totaux, ou HCT, ou mesure en mode HCT), et le détecteur émet un signal ST représentatif de la quantité totale d'hydrocarbures. Dans le premier cas, seul le méthane parvient au détecteur et celui-ci émet un signal S4 représentatif de la quantité totale de méthane. Un traitement des signaux, par exemple à l'aide de moyens numériques de traitement de signaux et de calcul 7, permet ensuite, par soustraction ST - S4, de déduire une mesure de l'ensemble des hydrocarbures gazeux non méthane.

Un catalyseur pouvant être utilisé pour la combustion des hydrocarbures gazeux non méthane peut être :
- un oxyde métallique ou un mélange d'oxydes métalliques déposés sur un absorbant de type zéolithe (alumine ou autre...). L'oxyde métallique peut être: du MnO2(oxyde de manganèse), du CuO et Cu2O,(oxyde de cuivre), du ZrO2 (oxyde de zirconium)...
- un métal précieux ou un mélange de métaux précieux déposés sur un adsorbant de type zéolithe (alumine ou autre...). Le métal peut être par exemple : Pt (platine), ou Ni (nickel), ou Rh (rhodium).

On peut utiliser aussi de la toile, de la mousse ou du fil de Pt, de Ni. Un autre exemple de catalyseur est la Carulite, de composition : 60% à 75% de MnO2, de 11% à 14% de CuO, de 15% à 16% de Al2O3. Encore un autre exemple de catalyseur est l'hopcalite, de composition 33 - 44 % MnO2, 22 - 36 % CuO (le reste étant un liant).

Le détecteur 8 est par exemple un détecteur à ionisation de flamme. Dans ce cas, de l'hydrogène est en outre mélangé au gaz à analyser à l'entrée du détecteur 8. De l'air est injecté au-dessus de la flamme d'hydrogène et de gaz à analyser, cet air servant à évacuer la vapeur d'eau formée par la combustion.

Un exemple d'un tel détecteur est celui vendu par la société Environnement S.A. (111, Bd Robespierre, 78300 POISSY, FRANCE) sous la référence HC51M.

Le débit d'hydrogène est de préférence tel que la composition du mélange hydrogène-gaz à analyser (oxygène) est compris entre 10 % et 40 % ou est proche ou est de l'ordre du rapport O2 - H2 dans l'air (environ 30%). Ceci permet au détecteur de pouvoir fonctionner avec un débit d'échantillon d'oxygène tout en obtenant la même sensibilité de détection que pour des tests effectués sur l'air.

Une augmentation du débit d'hydrogène a sans doute aussi pour effet de modifier la forme de la flamme où est collecté le courant ionique produit par la combustion des HCnM. L'électrode collectrice de ce courant est positionnée au-dessus de la flamme et la proportion d'ions collectés peut être différente en fonction de la forme de la flamme.

A titre d'exemple, le débit d'hydrogène est d'environ 130 ml/min (contre 40 ml/min pour une utilisation dans de l'air), les débits d'échantillon (oxygène) et d'air (purge ou évacuation de la vapeur d'eau) étant respectivement de 80 ml/min et de 400 ml/min.

La température du catalyseur 6 est de préférence choisie telle que le moins possible de CH4 soit brûlé. En effet, dans le cas de la carulite, pour une température d'environ 210°C, on a pu constater qu'une proportion non négligeable de CH4 était brûlée. Or, le calcul de la concentration des HCnM consiste à faire la différence entre la mesure en mode HCT (lorsque l'échantillon passe directement dans le détecteur) et la mesure en mode CH4 (lorsque l'échantillon passe dans le catalyseur avant d'aller dans le détecteur). La concentration en HCnM est alors majorée d'une façon importante.

Afin de réduire la combustion du méthane dans le catalyseur, la température a été ajustée en deux étapes (cas de la carulite):
1. Réglage de la température à 163°C. L'analyse d'un mélange à 10 ppm de CH4 + 1 ppm de C2H6 dans de l'oxygène fonctionne bien, mais l'analyse d'un mélange à 52.2 ppm de C2H6 + 52.5 ppm de CH4 dans l'oxygène montre qu'environ 10 % de C2H6 n'est pas brûlé, proportion qu'on ne peut pas évaluer avec précision sur une teneur en C2H6 de 1 ppm.
2. Réglage de la température à 182 °C . Les résultats sont satisfaisants sur les 2 mélanges précédents et sur un mélange qui ne contient pas de CH4, mais uniquement 8 ppm de C2H6 dans de l'oxygène pour lequel tout le C2H6 est brûlé.

Le réglage de la température de fonctionnement du convertisseur d'hydrocarbures non méthanes, par exemple entre 160°C et 190°C, permet donc de ne pas perdre de méthane (ou d'en perdre au maximum quelques %, par exemple au maximum 3% ou 5%) tout en convertissant les hydrocarbures non méthanes (HCnM) de l'échantillon d'oxygène à analyser.

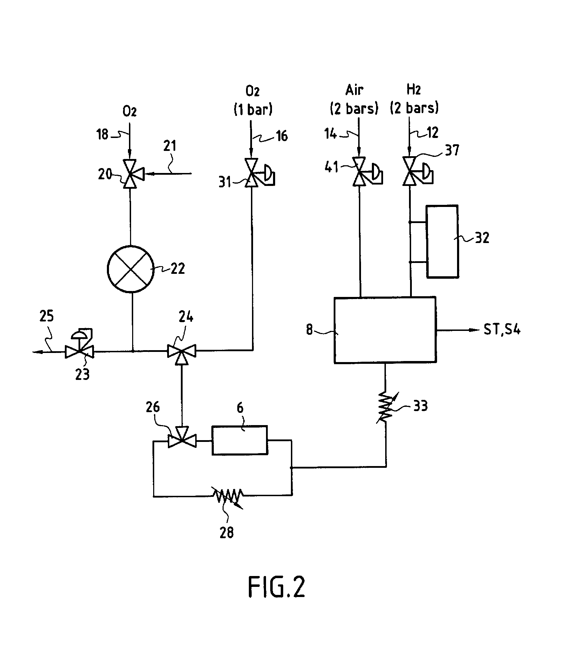

La figure 2 représente un exemple détaillé du système de détection 10 avec un détecteur, de type HC51M déjà mentionné ci-dessus, tel qu'utilisé dans le cadre de la présente invention. Ce système met en oeuvre un détecteur 8 à ionisation de flamme.

Comme déjà expliqué ci-dessus, de l'hydrogène et de l'air sont introduits dans ce détecteur, respectivement par les voies 12 et 14.

De l'oxygène, à pression d'environ un bar, est introduit par la voie 16, afin d'établir le zéro de l'appareil.

L'oxygène à analyser est introduit par la voie 18, la voie 21 permettant d'introduire un gaz étalon à pression atmosphérique.

La voie 25 permet de purger un excès d'échantillons introduit dans le circuit.

De manière plus détaillée, la voie 12 comporte successivement un régulateur 37, aux bornes duquel une boucle d'allumage 32 est disposée.

La voie 14 comporte successivement un épurateur (non représenté) et un régulateur 41.

La voie 16 comporte un épurateur (non représenté), un régulateur 31, et se raccorde à une électrovanne 24 à trois voies.

Par cette électrovanne 24 arrive également l'échantillon de gaz à analyser, une vanne 20 et une pompe 22. Un excès d'échantillon est purgé par l'intermédiaire d'un régulateur 23.

Une vanne à trois voies 26 permet d'envoyer un gaz à analyser, soit directement vers l'analyseur 30 (la référence 28 désigne un capillaire d'équilibrage de charge), soit à un catalyseur 6 afin de brûler les hydrocarbures non méthane. La référence 33 désigne un capillaire variable, permettant de régler le débit en entrée du détecteur 30. Ce dernier délivre des signaux ST et S4, à partir desquels un calculateur peut calculer, par soustraction, un signal représentatif de la quantité d'hydrocarbures non méthane.

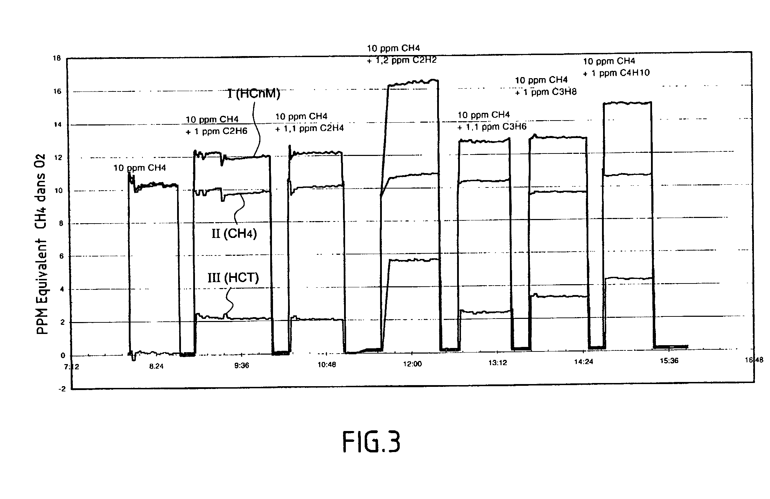

La figure 3 représente un test de réponse des hydrocarbures non méthanes (HCnM) en Cn (n = 2, 3, 4) dans de l'oxygène en présence de 10 ppm de CH4. Les essais concernent :
- 10 ppm CH4 dans O2
- 10 ppm CH4 + 1 ppm de C2H6 dans O2
- 10 ppm CH4 + 1,1 ppm de C2H4 dans O2
- 10 ppm CH4 + 1,2 ppm de C2H2 dans O2
- 10 ppm CH4 + 1,1 ppm de C3H6 dans O2
- 10 ppm CH4 + 1,1 ppm de C3H8 dans O2
- 10 ppm CH4 + 1 ppm de C4H10 dans O2

L'appareil utilisé est du type vendu par la société Environnement S.A. (111, Bd Robespierre, 78300 POISSY, FRANCE) sous la référence HC51M, avec les conditions suivantes de fonctionnement :
- température de catalyseur : 182°C,
- débit d'hydrogène : environ 130 ml/min,
- débit d'échantillon : 80 ml/min
- débit d'air comburant : 400 ml/min
- catalyseur : carulite (composition indiquée ci-dessus).

La courbe I donne la quantité de HCnM, la courbe II la quantité de CH4, la courbe III la quantité totale d'hydrocarbures.

Ces essais indiquent que la réponse du détecteur à ionisation de flamme est bien proportionnelle au nombre d'atomes de carbone du CnHm à mesurer sauf pour le C2H2 (le C2H2 a une meilleure réponse). L'appareil est capable de détecter moins de 1 ppm de CnHm en équivalent CH4 dans de l'oxygène contenant 10 ppm de CH4. Soit moins de 0.5 ppm de C2, de 0.3 ppm de C3 et 0.25 ppm de C4.

Ce test donne des résultats similaires ou identiques en sensibilité à un test effectué pour des mesures de CnHm dans l'air (mais avec des conditions différentes: température de catalyseur de 210°C, débit d'hydrogène de 40 ml/min, débit d'échantillon de 80 ml/min, débit d'air comburant de 400 ml/min).

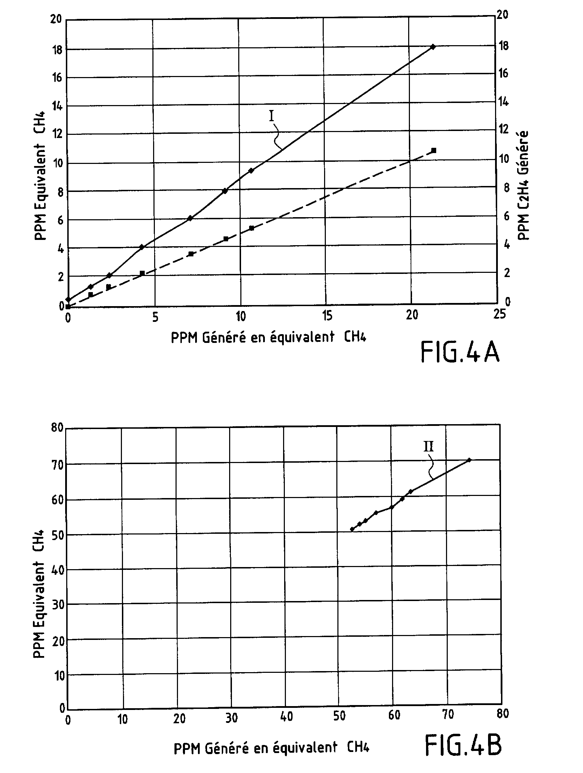

Sur les figures 4A à 6B, la courbe I représente l'évolution de la concentration en HCnM, en équivalent CH4, et la courbe II l'évolution de la concentration en HCT, également mesurée en équivalent CH4.

Les figures 4A et 4B représentent la réponse de la dilution d'un mélange de HCnM à 10.7 ppm de C2H4 dans de l'oxygène contenant 52.9 ppm de CH4 avec de l'oxygène contenant 52.7 ppm de CH4 (ceci permet de diluer progressivement le mélange de HCnM, donc de faire varier la concentration de C2H4, tout en gardant une concentration constante de CH4). Cet essai, et notamment la courbe I, montre qu'il est possible d'estimer un seuil de détection de HCnM en dessous de 5 ppm en équivalent CH4, soit environ moins de 2 à 3 ppm de C2H4.

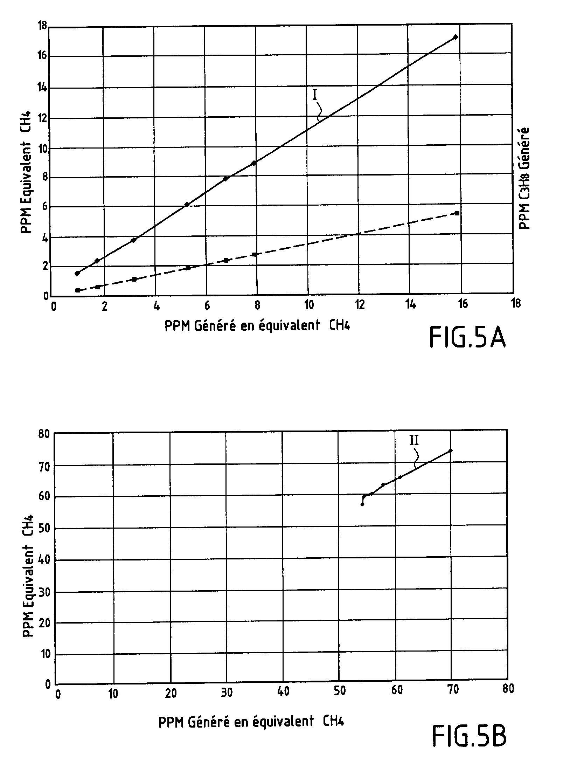

Les figures 5A et 5B représentent la réponse de la dilution d'un mélange de HCnM à 5.3 ppm de C3H8 dans de l'oxygène contenant 49.7 ppm de CH4 avec de l'oxygène contenant 52.7 ppm de CH4. Cet essai montre comme précédemment une détection de HCnM à moins de 5 ppm en équivalent CH4, soit, en C3H8, moins de 2 ppm.

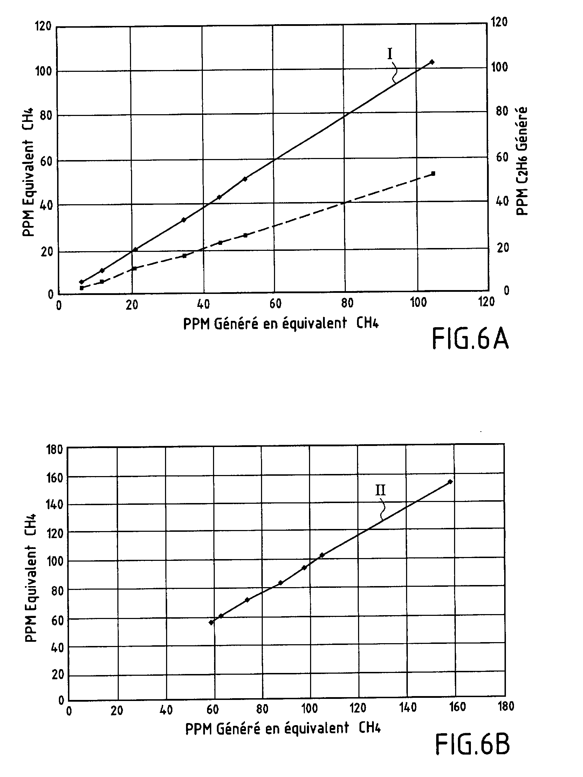

Les figures 6A et 6B représentent la réponse de la dilution d'un mélange de HCnM à 52.2 ppm de C2H6 dans de l'oxygène contenant 52.5 ppm de CH4 avec un mélange O2 contenant 52.7 ppm de CH4. Cet essai confirme les résultats précédents et permet de vérifier la linéarité de la réponse de l'appareil de 0 à 160 ppm en équivalent CH4.

Dans les 3 exemples donnés ci-dessus, la courbe I montre qu'il est possible, selon l'invention, de détecter moins de 5 ppm d'hydrocarbures non méthane (en équivalent CH4), et ceci dans environ 50 ppm de méthane.

Suite à ces essais, la dérive de l'appareil sur la mesure faite sur de l'oxygène du circuit de zéro (oxygène circulant par la voie 16 du schéma de la figure 2) a été mesurée. Sur une vingtaine d'heures, la dérive en HCnM est inférieure à 0.1 ppm.

On notera que des impuretés peuvent empoisonner le catalyseur lorsqu'il est utilisé directement sur de l'air ambiant comme gaz à analyser. Au contraire, de telles impuretés n'existent pas lorsque de l'oxygène est utilisé comme gaz à analyser, comme dans le cadre de la présente invention (et notamment de l'oxygène issu d'une unité de production de gaz de l'air). L'utilisation selon la présente invention permet donc d'augmenter la durée d'utilisation des catalyseurs.

Selon un exemple de résultat l'invention permet de détecter moins de 5 ppm d'hydrocarbures en équivalent méthane, dans de l'oxygène contenant environ 50 ppm de Méthane (voir figures 4A à 6B commentées ci-dessus), soit moins de 2 à 3 ppm d'HydroCarbures non Méthanes en C2, moins de 2 ppm en C3 et moins de 1 ppm en C4.

Selon un autre exemple de résultat, l'invention permet de détecter moins de 1 ppm en équivalent CH4 de HCnM (de type C2, et/ou C3, et/ou C4), notamment dans de l'oxygène contenant 10 ppm de CH4 (voir figure 3 et commentaire correspondant ci-dessus), soit moins de 0,5 ppm de C2H6, 0,3 ppm de C3H8 et 0,25 ppm de C4H10.

Selon encore un autre exemple de résultat, l'invention permet de détecter moins de 5 ppm en équivalent CH4 de HCnM (C2, et/ou C3), notamment dans de l'oxygène contenant 50 ppm de CH4 (voir figures 4A - 6B commentées ci-dessus) , soit moins de 2 à 3 ppm de C2 et moins de 2 ppm de C3.

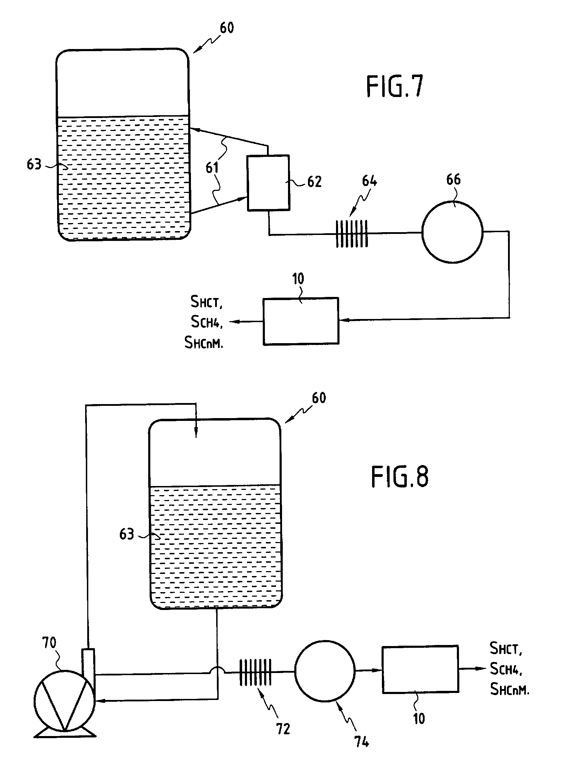

Un dispositif et un procédé tels que décrits ci-dessus peuvent être utilisés dans une unité de production de gaz de l'air. Un exemple d'une telle utilisation est illustrée en figures 7 et 8.

Sur ces figures la référence 60 désigne un réservoir d'oxygène liquide contenant un bain d'oxygène liquide 63. Un prélèvement d'oxygène liquide dans ce bain est réalisé par une tuyauterie d'une pompe 70 de remontée de liquide (figure 8) ou sur une prise 61 de type ascenseur (figure 7). Cette deuxième solution comprend une circulation de liquide vers un petit réservoir 62 situé près de la paroi du réservoir 60. Il y a ensuite vaporisation intégrale du liquide dans un vaporisateur constitué par un capillaire et un échangeur atmosphérique 64, 72. Les constituants du mélange gazeux à analyser sont ensuite homogénéisés à l'aide d'un mélangeur 66, 74. Le débit de l'échantillon gazeux est par exemple d'environ 0.5 à 1 Nm³/h. Ces deux dispositifs permettent d'échantillonner et d'acheminer rapidement, vers l'analyseur 10 d'hydrocarbures, un échantillon gazeux représentatif du liquide du bain 63 de l'unité de production. Ils permettent également de prélever un liquide souvent renouvelé : en effet, la composition du liquide contenu dans le réservoir 60, et notamment les impuretés qui sont à mesurer avec le dispositif selon l'invention, peut varier dans le temps. Il est donc préférable d'avoir à tout instant, dans le circuit de prélèvement, un échantillon qui représente, de manière aussi exacte que possible, la composition, au même instant, du liquide dans le réservoir 60. Ceci est rendu ici possible par l'utilisation de moyens tels que la pompe 70 de remontée de liquide ou la prise 61 de type ascenseur.

Un système et un procédé selon l'invention, tels que par exemple décrits ci-dessus en liaison avec les figures 1 ou 2 ou 7 ou 8, permettent de contrôler le taux d'hydrocarbures non méthanes dans de l'oxygène, et notamment dans un bain d'oxygène liquide, tel que le bain des vaporisateurs des unités de production de gaz de l'air. Lorsque le taux ou la concentration d'hydrocarbures non méthane dépasse une certaine valeur limite (qui, on le comprend d'après les figures 3 à 6C, peut être de l'ordre de quelques ppm en équivalent méthane, par exemple 5 ppm équivalent méthane ou moins, par exemple encore 1 ppm équivalent méthane) une alarme peut être déclenchée, et un risque d'explosion est ainsi évité ou réduit. Les valeurs limites peuvent par exemple être définies dans les consignes d'exploitation des unités de production ou être stockées en mémoire dans l'unité de traitement 7 (voir figure 1) qui procède à la comparaison valeurs mesurées - valeurs limites.

Dans le cas d'une unité de production des gaz de l'air, les mesures continues de l'ensemble des hydrocarbures, du méthane et des hydrocarbures non méthane, permettent, en cas de dépassement de concentrations consignées en hydrocarbures non méthane, de procéder au déclenchement de procédures de mise en sécurité de l'unité de production. Par exemple, en fonction des niveaux des alarmes, il peut y avoir action sur le fonctionnement de l'épuration de l'air entrant, et/ou action sur le fonctionnement de la production et/ou arrêt de la production.

## Revendications

1. Procédé de détection d'hydrocarbures autres que le méthane dans un gaz comportant majoritairement ou essentiellement de l'oxygène, ainsi que du méthane et lesdits hydrocarbures autres que le méthane, ledit procédé comportant :
- une étape de détection de l'ensemble des hydrocarbures dans ledit gaz, fournissant une première valeur d'ensemble d'hydrocarbures,
- une étape de combustion des hydrocarbures autres que le méthane,
- une étape de détection du méthane dans ledit gaz, fournissant une deuxième valeur,
- une étape de calcul de la quantité d'hydrocarbures autres que le méthane par différence entre la première valeur et la deuxième valeur.

2. Procédé selon la revendication 1, ledit gaz comportant au moins 95 %, de préférence au moins 99% ou 99,5% d'oxygène.

3. Procédé selon la revendication 1 ou 2, ledit gaz comportant majoritairement ou essentiellement de l'oxygène, du méthane et des hydrocarbures autres que le méthane, lesdits hydrocarbures autres que le méthane étant présents, par rapport au méthane, dans une proportion de l'ordre de quelques pour cent.

4. Procédé selon la revendication 3, lesdits hydrocarbures autres que le méthane étant présents, par rapport au méthane, dans une proportion inférieure à 6% ou à 5 % ou à 4% ou à 3%.

5. Procédé selon la revendication 3 ou 4, ledit gaz comportant moins de 50 ppm de méthane.

6. Procédé selon l'une des revendications 3 à 5, lesdits hydrocarbures autres que le méthane étant présents à une concentration de moins de 5 ppm dans l'oxygène.

7. Procédé selon l'une des revendications 1 à 6, les hydrocarbures autres que le méthane étant brûlés par un catalyseur (6).

8. Procédé selon la revendication 7, la détection étant réalisée par un détecteur à ionisation de flamme (8).

9. Procédé selon la revendication 7 ou 8, de l'hydrogène étant mélangé au gaz à analyser, de sorte que le rapport hydrogène/oxygène soit compris entre 10% et 40 %.

10. Procédé selon l'une des revendications 7 à 9, dans lequel la température du catalyseur est telle que moins de 5% du méthane contenu dans le gaz est brûlé.

11. Procédé selon la revendication 10, la température du catalyseur étant comprise entre 160° C et 190° C.

12. Procédé de détection d'hydrocarbures autres que le méthane dans un bain (63) d'oxygène liquide d'un vaporisateur d'une unité de production de gaz de l'air, comportant :
- un prélèvement d'oxygène liquide dans ledit bain (63),
- une vaporisation dudit oxygène liquide, produisant un gaz vaporisé,
- un procédé de détection d'hydrocarbures autres que le méthane, dans ledit gaz vaporisé, selon l'une des revendications 1 à 11.

13. Procédé selon la revendication 12, le prélèvement étant réalisé à l'aide d'une tuyauterie d'une pompe (70) de remontée de liquide ou sur une prise (61) ascenseur.

14. Procédé selon l'une des revendications 12 ou 13, comportant en outre une étape de déclenchement d'une alarme lorsque la concentration ou le taux d'hydrocarbures autres que le méthane, dans ledit gaz vaporisé, dépasse une certaine valeur limite.

15. Dispositif de détection d'hydrocarbures autres que le méthane dans un gaz comportant majoritairement ou essentiellement de l'oxygène, ainsi que du méthane et lesdits hydrocarbures autres que le méthane, ledit dispositif comportant :
- des moyens (8) de détection de l'ensemble des hydrocarbures dans ledit gaz, fournissant une première valeur d'ensemble d'hydrocarbures,
- des moyens (6) de combustion des hydrocarbures autres que le méthane,
- des moyens (8) de détection du méthane, fournissant une deuxième valeur,
- des moyens (7) pour calculer la quantité d'hydrocarbures autres que le méthane, par différence entre la première valeur et la deuxième valeur.

16. Dispositif selon la revendication 15, les moyens de combustion des hydrocarbures autres que le méthane comportant un catalyseur (6).

17. Dispositif selon la revendication 15 ou 16, les moyens de détection de l'ensemble des hydrocarbures et les moyens de détection du méthane comportant un détecteur (8) à ionisation de flamme.

18. Dispositif de détection d'hydrocarbures autres que le méthane dans un bain d'oxygène liquide d'un vaporisateur d'une unité de fabrication de gaz de l'air, comportant :
- des moyens (61,62,70) de prélèvement d'oxygène liquide dans ledit bain,
- des moyens (64,72) pour vaporiser ledit oxygène liquide, produisant un gaz vaporisé,
- un dispositif (10) de détection selon l'une des revendications 15 à 17.

19. Dispositif de détection selon la revendication 18, comportant en outre des moyens pour déclencher une alarme lorsque la concentration ou le taux d'hydrocarbures autres que le méthane, dans ledit gaz vaporisé, dépasse une certaine valeur limite.
